(19) Europäisches Patentamt

European Patent Office

Office européen des brevets

(11) **EP 0 578 756 B1**

(12) **EUROPEAN PATENT SPECIFICATION**

(45) Date of publication and mention
of the grant of the patent:
**30.08.2000 Bulletin 2000/35**

(51) Int. Cl.[7]: **A61F 9/00**, A61B 18/00

(86) International application number:
**PCT/US92/02871**

(21) Application number: **92910340.6**

(22) Date of filing: **03.04.1992**

(87) International publication number:
**WO 92/17138 (15.10.1992 Gazette 1992/26)**

(54) **LASER SURGICAL PROBE**

CHIRURGISCHE LASERSONDE

SONDE CHIRURGICALE A FAISCEAU LASER

(84) Designated Contracting States:
**AT BE CH DE DK ES FR GB GR IT LI LU MC NL SE**

(30) Priority: **04.04.1991 US 681318**
**04.04.1991 US 680815**

(43) Date of publication of application:
**19.01.1994 Bulletin 1994/03**

(73) Proprietor:
**PREMIER LASER SYSTEMS, INC.**
**Irvine, California 92718 (US)**

(72) Inventors:
• **COLVARD, Michael, D.**
**Pacific Palisades, CA 90272 (US)**
• **COZEAN, Colette**
**El Toro, CA 92630 (US)**
• **AMIRKHANIAN, Barouj, D.**
**Glendale, CA 91203 (US)**

(74) Representative:
**Whitaker, Iain Mark et al**
**Sommerville & Rushton**
**Business Link Building**
**45 Grosvenor Road**
**St. Albans, Hertfordshire AL1 3AW (GB)**

(56) References cited:
**EP-A- 0 214 712**      **EP-A- 0 293 126**
**EP-A- 0 392 951**      **WO-A-89/03202**
**US-A- 4 597 380**      **US-A- 4 625 724**
**US-A- 4 718 417**      **US-A- 4 740 047**

**Description**

Background of the Invention

The present invention relates generally to the field of laser surgical probes. More specifically, the present invention relates to laser surgical probes in which laser energy is output generally transversely relative to the laser energy input to the probe.

Surgical techniques making use of laser technology have been developed for a variety of procedures. However, the usefulness of standard surgical laser probes is limited in many of these procedures, such as where the surgeon must operate within a tightly confined body cavity or lumen, or where the area requiring laser treatment is accessible only around a tight corner.

One type of surgery which has heretofore not employed laser technology involves anterior capsulotomies. In ophthalmic surgery, it is frequently necessary to perform these procedures in order to expose a portion of the lens underlying the anterior capsule. One example where anterior capsulotomies are useful is where a surgeon desires to remove all or part of the natural lens for replacement with an intraocular lens (IOL).

A number of techniques for anterior capsulotomy have been developed, many of which can be classified as "can opener" techniques, in which small scores are first placed around the region of the anterior capsule to be removed. These scores can be made by any of a variety of techniques, including the use of a needle, vibrating needle, or photodisruptive laser. After scoring, the surgeon tears between each of the scores to create a serrated capsular margin.

A disadvantage of can opener techniques for anterior capsulotomy is that unintended extension of the tears between scores can occur to form eccentric radial tears. These radial tears have been shown to result in asymmetric forces upon capsular contraction during healing. These asymmetric forces can result in decentration of an implanted IOL in the direction of the radial tear.

An improvement on can opener techniques for anterior capsulotomy is the continuous tear capsulotomy technique. This technique requires that the surgeon continuously tear the opening in the capsule. The technique results in a smooth non-serrated capsulotomy margin which is quite resistant to unplanned radial tears. However, the technique requires great skill, and the average ophthalmic surgeon is not likely to master the technique without extensive training and experience.

Various types of laser probes may be found in the prior art for ophthalmic and other uses, particularly endoscopic uses. For example, PCT Publication No WO-A-85/03202 discloses a probe for laser emulsification of cataracts or the like. Laser light is delivered through an optical fibre to tips of various configurations. Similarly, US Patent No: 4,740,047 discloses a probe that can be used for endoscopic applications that require a lateral beam output.

While laser based surgical devices are known from the prior art, these devices are generally not suited for some types of surgeries, such as anterior capsulotomy, that impose specific requirements on the laser surgical device. For example, US Patent No: 4,597,380 which is considered by the Applicants to be the closest prior art and over which the present invention is characterised, discloses an endoscopic attachment to a surgical laser which has an endoscopic tube adapted for lasing and viewing of the working area longitudinally ahead of the tube and has a directionable reflecting mirror which allows directing of the laser beam while viewing the working area. Further, US Patent No: 4,625,724 discloses a laser vascular anastomosis apparatus which comprises a probe that is positioned within vascular ducts that are then irradiated with lasers emanating from the tip of the probe. US Patent No: 4,718,417 discloses a diagnostic tool used in laser angiosurgery which is not suited for performing actual surgery, especially surgery in confined spaces such as anterior capsulotomies.

Summary of the Invention

According to the present invention there is provided a laser surgery probe as defined in claim 1.

In a preferred form of the probe, said inclined surface is formed on an end of a sapphire rod, which is generally wedge-shaped.

In one realisation of the present invention, there is provided an optical apparatus for a laser surgical probe which receives a laser light beam along a longitudinal axis. The optical apparatus comprises a transparent reflecting surface which redirects the light beam at an angle to the longitudinal axis, and a collimator for collimating the light beam such that light incident on the reflecting surface is collimated, preferably before it has been redirected by the reflecting surface. The reflecting surface is preferably at an angle relative to the longitudinal axis at least as great as the critical angle for total internal reflection of the laser light. The reflecting surface preferably lies on a rod of dielectric material. Light is preferably delivered via an optical fibre, with the collimator comprising a sapphire microball having its centre at the focal distance from the proximal end of the fibre. The optical apparatus preferably also includes a disposable, single-use optical fibre, preferably fluoride-based, having a proximal end and a distal end. The optical apparatus is preferably removable from the probe so as to expose the distal end of said fibre.

In another realisation of the present invention, there is provided a laser surgical probe, comprising a flexible waveguide for directing a laser light beam along a longitudinal axis, a rod which receives the laser light beam along the longitudinal axis, the rod having a wedge at its distal end for manipulation of tissue, and a transparent reflecting surface at its distal end, the reflecting surface serving to redirect the laser light beam at an angle relative to the longitudinal axis. The probe preferably has an output surface on the rod where the light beam exits the rod, and also has a housing with the wedge extending distally beyond the housing. The reflecting surface is at an angle relative to the longitudinal axis at least as great as the critical angle required for total internal reflection of the laser light.

The probe of the present invention may be used for performing a surgical procedure on tissue in a mammal, in which case the probe is inserted into an internal portion, such as the anterior chamber of the eye, of the mammal, a laser light beam is directed into the probe along the longitudinal axis, and is redirected so that the beam exits the probe along an output axis which is at an angle relative to the longitudinal axis of the probe and can be generally perpendicular to the longitudinal axis, towards the tissue, thereby the laser-tissue interaction can be seen through the probe. The procedure preferably also includes passing visible light through the probe such that the visible light propagates along a viewing axis which is generally parallel to or along the output axis, and viewing the visible light after passing through the probe. Preferably the laser light beam has a wavelength in an invisible portion of the optical spectrum and is at an absorbance peak of water. Light having a wavelength of 2.94 µm is especially preferred. Redirecting the laser light beam can be accomplished by reflecting the light beam at a surface of the probe through total internal reflection, with the surface being transparent to the visible light. A smooth non-serrated capsulotomy margin can be formed with the laser light beam, followed by phacoemulsification or phacoablation of the lens underlying the anterior capsule. Advantageously, phacoemulsification can be performed using laser light from the probe. For some procedures the tissue may be irrigated with irrigation fluid, such as BSS, and fluid aspirated from the region of the tissue, e.g. for a procedure on an eye, viscoelastic material, such as "Healon", may be applied to the eye to maintain patency of the eye and hold tissues in position.

A probe in accordance with the invention may also be used in performing an anterior capsulotomy in the eye of a mammal, in which case the laser probe is inserted into the anterior chamber of the eye, a laser light beam is directed into the laser probe along the longitudinal axis, and redirected at an angle relative to the axis, to form a smooth non-serrated capsulotomy margin on the anterior capsule of the eye. The inclined surface of the probe would be utilised to manipulate the portion of the anterior capsule within the margin for removal. Redirecting of the beam may be accomplished by totally internally reflecting the light beam at this surface of the probe. While forming the margin, the margin can be viewed by looking through a transparent portion of the probe. The laser light beam used for this procedure would preferably be of non-ultraviolet wavelength, more preferably also a wavelength at an absorbance peak of water. Such wavelengths can be produced by an Erbium:YAG laser. The light beam is also preferably provided as pulses at a frequency greater than 10 Hz, more preferably between 20 Hz and 30 Hz. The probe is preferably inserted through an incision in the sclera of the eye.

It will be appreciated that a probe in accordance with this invention can be used to perform surgical procedures involving cutting, phacoemulsification or phacoablation of tissue. It can also be used for:

ophthalmic procedures selected from the group consisting of corneal surgeries, keratectomy, keratoplasty, glaucoma surgeries, filtration procedures, trabeculoplasty, iridectomies, iridotomies, cataract surgeries, capsulotomy, cataract extraction, vitreous surgeries, cutting of the vitreous bands, retinal surgery, removal of retinal membrane, and repair of retinal tears, and for

non-ophthalmic procedures on mammals selected from the group consisting of surgery within a joint, surgery within a knee, procedures within long narrow passages, cardiovascular surgery and urethral surgery.

Further objects, features and other advantages of the present invention will become apparent from the ensuing detailed description, considered together with the appended drawings.

Brief Description of the Drawings

Figure 1 is a partially cut away, partially exploded, general perspective view of a laser probe,

Figure 2 is a partial cross-sectional view of one embodiment of the laser probe,

Figure 3 is a cross-sectional view of another embodiment of the laser probe,

Figure 3a is a cross-sectional view of a modified laser probe of Figure 3.

Figure 3b is a perspective view of the rod used in the modified laser probe of Figure 3a,

Figure 4 is a partial cross-sectional view in perspective of an eye of a mammal showing the laser probe in use during an anterior capsulotomy procedure, and

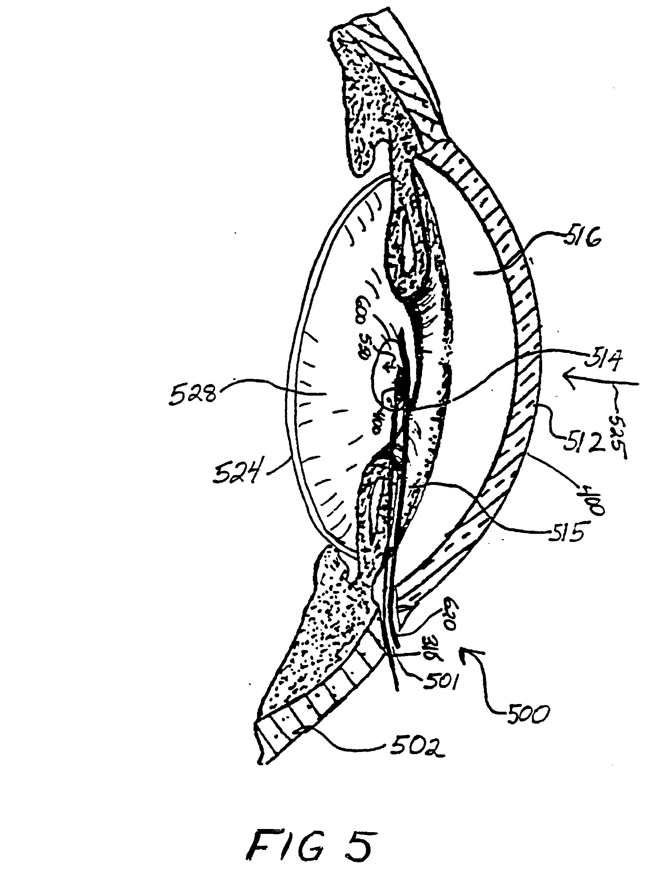

Figure 5 is a partial cross-sectional view in perspective of an eye of a mammal showing the laser probe in use during a phacoemulsification procedure with irrigation.

Detailed Description of the Preferred Embodiment

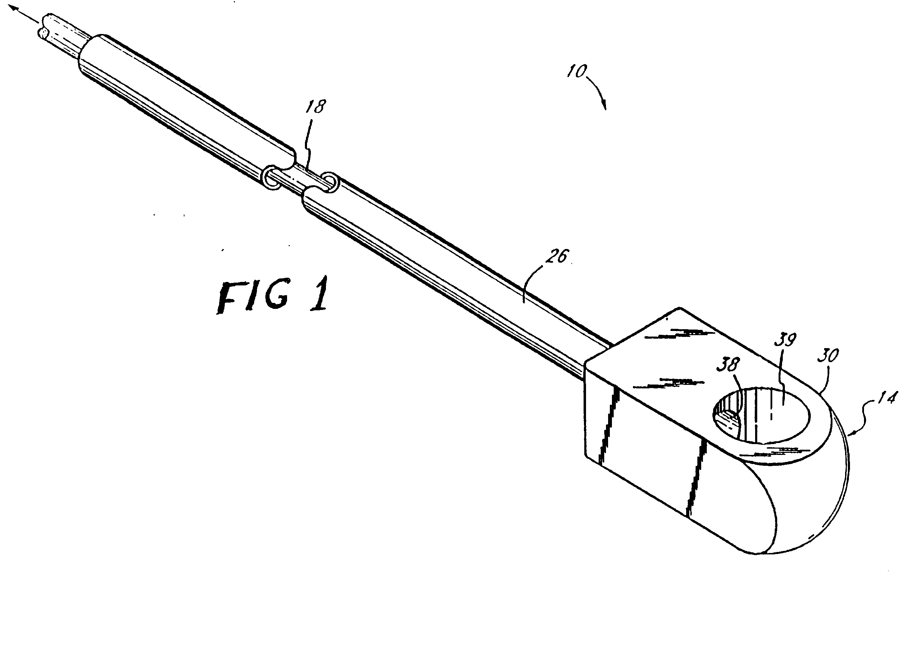

Referring now to the drawings in detail, wherein like reference numerals designate like elements throughout the several views thereof, there is shown generally at 10 in Figure 1, a laser surgical probe suitable for use with the present invention. The probe 10 comprises an elongate housing 14 and an optical fiber 18 for transmitting laser light to optical apparatus to be described below located within the housing 14, which housing is preferably no more than 2.5 mm.

Referring to Figure 1 the housing 14 comprises a fiber holder comprising an axially elongate hollow shaft, 26 and a head element 30 at the distal end of the shaft 26. The term "distal" designates the direction away from the laser light source, to which the probe is optically coupled. The tern, "proximal" shall mean the direction toward the laser light source 34. The term "longitudinal" shall be used to refer to a direction corresponding to an imaginary line running between proximal and distal ends. In Figure 1, a portion of the shaft 26 is cut away to reveal the fiber 18 extending therethrough.

The head element 30 is contoured for smooth insertion into interior portions of a mammal. In order to allow the head element 30 to be withdrawn from the mammal without snagging, the head element is generally symmetrical about the axis for insertion. The head element 30 is also smoothly contoured at its proximal and distal ends in order to prevent snagging upon insertion or withdrawal of the probe 10. As an alternative, the entire probe 10 can be housed within an outer housing (not shown) which can be contoured for smooth insertion and withdrawal without snagging.

The head element 30 is preferably constructed from metal, such as aluminium or stainless steel. The head element 30 has a hollow space including a longitudinal tubular cavity 38 and a transverse tubular cavity 39, which allow for insertion of the optical fiber 18 and said optical apparatus. The transverse tubular cavity 39 extends through the head element 30 to form top and bottom openings in the head element 30.

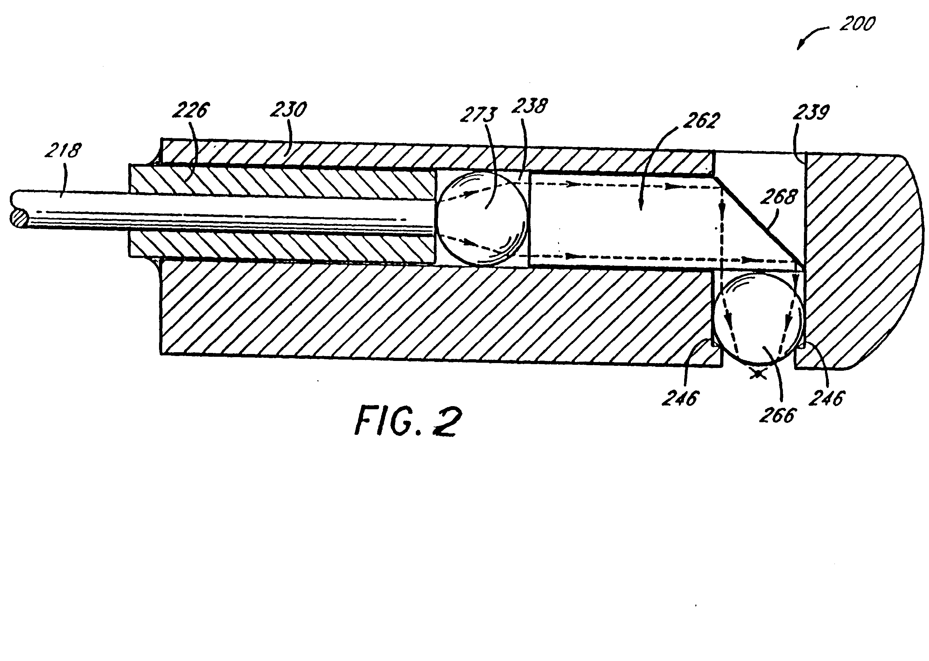

Referring now to Figure 2, there is shown one embodiment of a laser surgical probe 200. The probe 200 comprises an optical fiber 218 within a fiber holder shaft 226 and a head element 230. As discussed above the head element 230 is contoured for smooth insertion and withdrawal and has a longitudinal tubular cavity 238 and a transverse cavity 239. In this embodiment, the diameter of the transverse cavity 239 is constricted at the bottom relative to the remaining portion of the cavity 239, which is substantially tubular. Thus, a ledge 246 is formed within the transverse cavity 239.

The optical apparatus of this embodiment comprises separate pieces, namely a collimator 273, a diverter 262 and an intensifier 266. The collimator 273 and the intensifier portion 266 comprise microballs preferably formed from dielectric material, each having a spherical surface for refracting light optionally coated with an anti-reflective coating.

The diverter 262 preferably comprises a sapphire rod polished at a 45° angle at its distal end. The angled portion is coated with a reflective coating and forms a reflecting surface 268. The diameter of the rod is slightly smaller than the diameter of the longitudinal cavity 238 so that the diverter can be inserted therethrough with its axis parallel with the longitudinal axis of the probe.

The intensifier microball 266 rests on the ledge 246 and the diverter 266 is inserted through the longitudinal cavity 238 such that the microball 266 is held in the space between the ledge 246 and the diverter portion 268. The collimator microball 273, like the diverter portion 266, has a diameter slightly smaller than the diameter of the longitudinal cavity 238, and is inserted proximally of the diverter portion 266. Finally the optical fiber 218 within the shaft 226 is inserted into the longitudinal cavity 238. The shaft 226 is held to the proximal end of the head element 230 with U.V. curable glue. Thus, the diverter portion 266 and The collimator microball 273 are held within the portion of the longitudinal cavity 239 distal of the shaft 226. Preferably, There is substantially no space between the intensifier microball 266 and the diverter 262 or between the diverter 262 and the collimator microball 273.

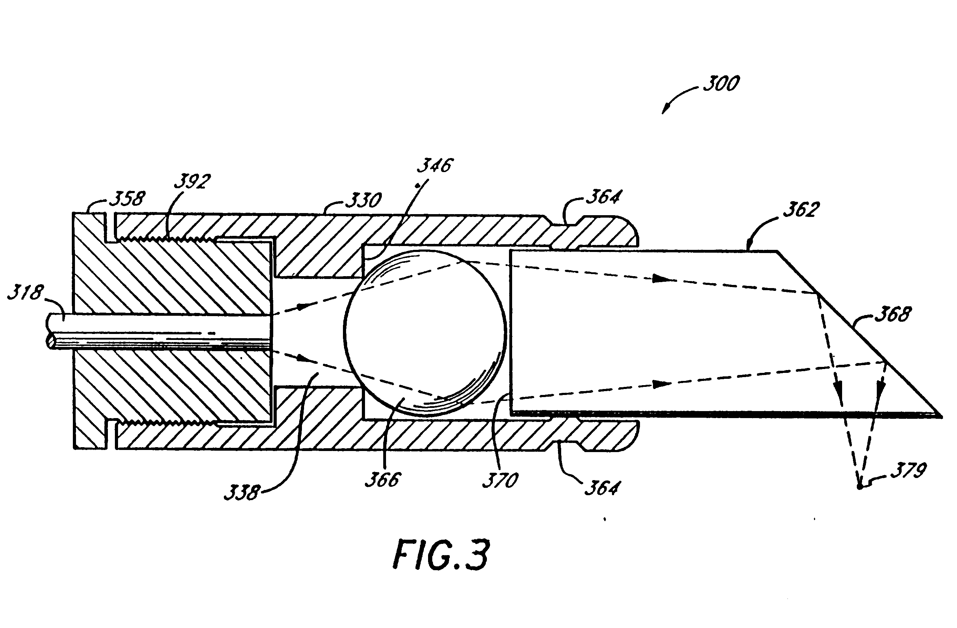

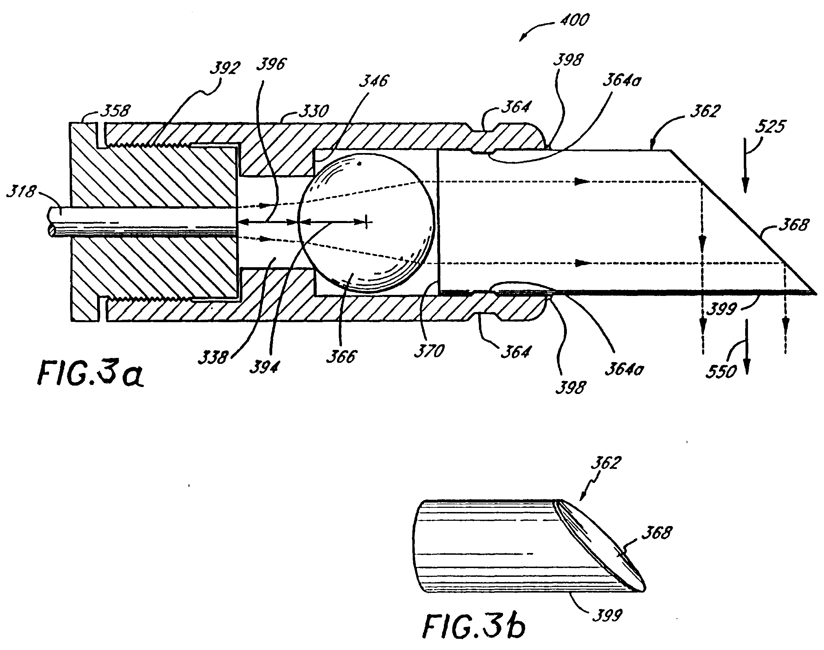

Referring now to Figure 3, there is shown another embodiment of a laser surgical probe 300. The probe 300 comprises an optical fiber 318, a head element 330 and a fiber holder 358. The fiber holder 358 serves to provide a grip for the operator of the probe 300 and also serves as a sleeve for the optical fiber 318.

The head element 330 has a tubular longitudinal cavity 338 having proximal, middle and distal sections. The middle section has a constricted diameter relative to the proximal and distal sections of the cavity 338. The proximal section of the cavity 338 is threaded to accept a Threaded portion 392 of the fiber holder 358.

The optical apparatus of the probe 300 comprises an intensifier 366 and a diverter 362. The intensifier comprises a spherical microball which is disposed in the distal section, and which rests on a ledge 346 formed by the constricted diameter of the middle section of the longitudinal cavity 338.

The diverter 362 comprises a sapphire rod polished at a 45° angle at its distal end. The angled portion is coated with a reflective coating to form a reflecting surface 368. The diameter of the rod is slightly smaller than the diameter of the longitudinal cavity 338 so that the diverter can be inserted therethrough. The diverter 362 can extend longitudinally beyond the distal end of the head element 330 as shown, or can be encased by the head element 330, with a hole at the point of emission of laser light energy. If the diverting portion extends beyond the distal end of the head element 330, the exposed sapphire can optionally be coated with a protective over-coat.

The distance between the distal end of the fiber 318 and the proximal portion of the intensifier microball 366, and the distance from the distal portion of the intensifier microball and the reflecting surface are selected so as to produce a focal point 379 a desired distance (e.g., less than 1 mm) from the bottom of the diverter 362. The distance from the intensifier microball 366 and diverter 362 can also be manipulated to provide the desired focal point 379. However, preferably, an input surface 370 of the diverter 362 is touching or almost touching the intensifier microball 366 in order to prevent axial movement of the microball 366.

The diverter 362 can be held in place by providing a notch 364 on the head element 330 and crimping the notch 364 to the diverter 362. If desired, the input surface 370 of the diverter 362 and the entire or proximal surface of the intensifier microball 366 may be coated with an anti-reflective coating to minimize reflection.

In use, the probe 300 is inserted into an internal portion of a mammal (e.g. an eye cavity) such that the rod 362 is surrounded by tissue and the portion of the rod extending from the housing is in contact with the tissue, although in the embodiment disclosed, the light beam is redirected by reflection, it will be understood that, by eliminating the reflective coating so that the light passes through the angled output face, redirection by refraction could be achieved. Such refraction is due to differences in refractive index at the angled output face. Redirection of the light beam by refraction may be similarly achieved by utilizing a bare rod, such as an optical fiber, and cleaving the end of the fiber of an angle (e.g. 45°) to cause the light output from the fiber to be deflected. Nevertheless, use of the reflective coating is preferred because a greater angle of deflection is possible.

Referring now to Figure 3a, there is shown a probe 400 that is a modification of the laser probe 300 of Figure 3. In this modified probe 400, the microball 366 is placed with its center 395 at its focal distance (f) from the distal end of the fiber 318. Placing the microball 366 in this position will achieve collimation of light incident thereon.

The focal distance (f) of a spherical lens can be calculated as $f = n \cdot r/2(n-1)$, where n is the index of refraction and R is the radius 394 of the lens. For the preferred embodiment having a 1.5 mm diameter spherical sapphire lens and using a laser producing light energy of wavelength 2.94 $\mu$m, the index of refraction of the lens is 1.72. Thus, for this preferred embodiment, f = 1.72(0.75)/2(1.72-1) = 0.896$\mu$m.

For a properly placed microball 366 achieving collimation of light from the fiber 318, the focal distance (f) will be equal to the length of the radius (R) 394 of the microball 366 plus the gap (g) 396 between the microball 366 and the distal end of the fiber 318, i.e. $f = g + R$. Thus, in the preferred embodiment described above, the proper gap 396 can be calculated as g = f - R = 0.896 mm - 0.75 mm = 0.146 mm .

In use of the probe 400, the collimated light travels along the longitudinal axis of the probe 400 until meeting the reflecting surface 368. The reflecting surface 368 diverts the light in a direction along an output axis 550 which is at an angle relative to the longitudinal axis of the probe 400. For many procedures, such as an anterior capsulotomy, the preferred angle of the output axis is perpendicular to the longitudinal axis of the probe 400. For other procedures, such as certain procedures in the treatment of glaucoma, angles greater or less than 90° are preferred.

In the modified laser probe 400, the reflecting surface 368 is transparent and requires no reflective coating. Rather, reflection occurs due to the total internal reflection achieved from the differences in the indices of refraction between the material of the rod 362 and the material surrounding the probe 400. In the preferred embodiment, the rod 362 is sapphire, having an index of refraction (n) of 1.7 with light produced by the Erbium:YAG laser having a wavelength of 2.94$\mu$m.

The critical angle ($\Theta_c$) of the reflecting surface 368 required to achieve total internal reflection can be determined according to Snell's law, which can be stated as follows: $n_1 \sin\Theta_1 = n_2 \sin\Theta_2$. As stated above, in the preferred embodiment, $n_1 = 1.7$. When the probe is used in air, $n_2 = 1.0$. For total internal reflection $\Theta_2 = 90°$. Thus, $\Theta_c$ for the rod of the preferred embodiment can be calculated from Snell's law as follows: $1.0\sin\Theta_c = 1.72\sin90°$. Accordingly, $\Theta_c = 35.5°$. This means that total internal reflection will be achieved as long as the reflecting surface 368 is at an angle greater than or equal to 35.5° with respect to the collimated light incident thereon.

The probe 400 functions to reflect light in substantially the same manner in a fluid environment (e.g. $H_2O$) as in air because the refractive index difference between the fluid and the sapphire rod 362 is sufficiently great to cause total internal reflection to occur at the surface 368.

As best seen in Figure 3 b, in the preferred embodiment, the rod 362 comprises a sapphire rod of circular cross section which is polished at a 45° angle at its distal end to form the reflecting surface 368. When the modified laser probe 400 of this preferred embodiment is used in air and not in contact with tissue, the curved bottom output surface 399 of the rod 362 acts as a cylindrical lens to focus the collimated light into a more linear form. However, when the laser probe 400 is used in a fluid environment (e.g. $H_2O$) or in contact with tissue, the lens action of the output surface 399 tends to be negated due to the higher index of refraction of the fluid and/or due to the contact with tissue at the cylindrical surface. Thus, when the modified laser probe 400 is used in internal portions of a mammalian body, such as in the eye, the light exiting the probe will be in a roughly tubular form. Advantageously, where total internal reflection is used to reflect the light within the probe 400 without the use of a reflective coating, the probe is transparent to allow the user of the probe 400 to view The point of contact of the laser light energy on tissues or other materials. This is particularly advantageous in surgeries, such as anterior capsulotomies, where the probe would otherwise obscure the contact point of the light energy. Another advantage of the probe 400 of Figure 3a is that the wedge-shaped end of the probe, formed by the angled reflecting surface 368, can be used as a tool to physically manipulate tissues without the need to insert an additional tool.

Although the 2.94 µm wavelength light energy of the Erbium:YAG laser is not visible to the human eye, the point of contact can generally be seen due to the energy released by the tissues after coming into contact with the laser energy from the probe 400. The tissue absorbs the collimated infrared light emitted from the probe along the output axis 550 and causes a laser-tissue interaction to occur. This interaction generally results in the release of light of a wide spectrum, including visible light, at many different angles. Thus, much of the light released by laser-tissue interaction will strike the reflecting surface 368 at an angle less than the critical angle. Even without release of significant quantities of light by laser-tissue interaction, the interaction can be seen by the formation of an incision or other effect on the tissue by the laser. Accordingly, the operator of the probe can look through the transparent reflecting surface 368 along a viewing axis 525 to view, the visible light from the tissue during operation of The probe 400. The viewing axis is generally perpendicular to the longitudinal axis of The probe 400.

In The preferred embodiment of the laser probe 400, all surfaces are polished with optical grade (e.g. 0.3 µm) polish, including the distal end of the fiber 318. Without this polishing, specular reflections can occur which result in a dispersal of the energy passing through the probe 400.

As discussed above, all surfaces should be generally smooth to prevent snagging during insertion or removal of the probe 400. Thus, in the preferred embodiment, one or more notches 364a are provided on the rod 362 which will allow the rod 362 to be held to the bead element 330 by crimping of the head element at the position of each notch 364a.

The laser probe 400 can advantageously be configured to supply irrigation fluid or vacuum for aspiration as frequently employed with known phacoemulsification devices. Alternatively, irrigation and/or aspiration can be supplied from separate devices inserted into the region of use of the probe 400.

In the preferred embodiment, it is important that fluid not enter the longitudinal cavity 338 of the head element 330 because the lenses are configured for use with air with an index of refraction of 1.0 in these spaces. The entry of fluid with a much higher index of refraction into the cavity 338 would prevent collimation of the light energy emanating from the fiber 318. Thus, a sealant 398, such as epoxy glue is preferably provided at the junction between the rod 362 and the head element 330, to prevent entry of fluid into the cavity 338.

The laser probes 10, 200, 300, 400 are useful in a wide variety of surgical procedures, including procedures such as described by Berlin in U.S. Patent No. 4,846,172. The use of the laser probes 10, 200, 300, 400 is especially advantageous in procedures where it is desired to operate a laser probe within a tightly confined space, such as within bodily tissues or a tightly confined body cavity or lumen. The probe allows a surgeon to direct laser energy from the side of the probe, thereby allowing laser energy to be directed around tight corners.

Particular examples of procedures in which the probe can be applied in a mammal include ophthalmic procedures of many types. In this regard, the probe can be used for cutting, phacoemulsification and phacoablation. These and other techniques using the probe are believed to be especially useful in corneal surgeries, such as keratectomy or keratoplastomy, in glaucoma surgeries, such as filtration procedures, trabeculoplasty, iridectomies or iridotomies, in cataract surgeries such as capsulotomy or cataract extraction, in vitreous surgeries such as cutting of the vitreous bands, and in retinal surgery such as removal of retinal membrane or repair of retinal tears. Non-ophthalmic procedures on a mammal in which the probe is believed to be useful include surgery within a joint, such as a knee, and procedures within long narrow passages, such as can be found within the cardiovascular system and the urethra.

The intensity of the light input to the probe is regulated, depending on the procedure, to provide sufficient intensity to achieve the desired result such as cutting, welding, vaporization or coagulation of biotic material (e.g. tissue). Where smooth cutting is desired, the frequency of the pulse should be in excess of 5 Hz, preferably 10 Hz - 30 Hz or more. It is also preferable to use a laser light source with a relatively low energy threshold in order to provide smooth cutting. Preferably the energy threshold is 5-10 mJ for cutting of the anterior capsule of the eye. Thus, for smooth cutting energy levels of 30 mJ per pulse are less are preferred, with energy levels just above the energy threshold of 5-10 mJ/pulse being especially preferred.

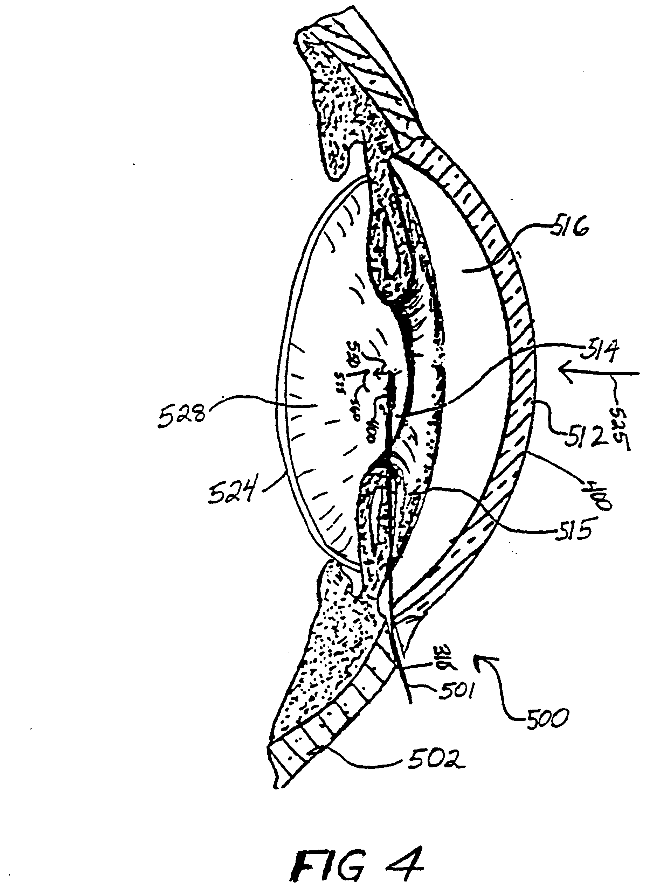

As stated above, one procedure in which the probe is particularly useful is in anterior capsulotomy of the eye. With reference to Figure 4, in which a cross section of an eye 510 is shown, a small roughly circular incision 501 through one side of the sclera 502 of the eye 500 is first made into the anterior chamber 516. This incision is roughly 2.0-3.5 mm in diameter. As discussed above, the probe is preferably contained within a housing having a diameter of 2.5 mm or less. This is advantageous in anterior capsulotomies and other procedures within the eye because larger probes would require a larger incision. Moreover, the use of large probes also increases the risk that the probe will come into contact the cornea 512, iris 515 or other delicate tissues within the eye 500, resulting in damage to these tissues.

In order to maintain the patency of the anterior chamber 516 during the procedure and to hold other tissues in position, the chamber 516 can be filled with a viscoelastic material, such as "Healon". The viscoelastic material will also hold tissues in position within the eye during the procedure. Alternatively, irrigation fluid, much as balanced salt solution (BSS) can be continuously infused to maintain patency of the chamber 516.

The probe 400 or other laser probe can be inserted into the incision 501 along with the fiber 318 transmitting laser energy thereto. The probe is then manipulated to cut a circular incision (shown partially formed at 560 in Figure 4 ) around a portion of the anterior capsule 524 adjacent the lens. As will be discussed in more detail below, the laser probe 400 advantageously allows the operator to view the energy released from the surface of the capsule as laser light energy is applied.

A variety of laser light sources ray be used in the procedure. However, it is preferred that the laser light source provide a smooth, non-serrated capsular margin, in order to enable a surgeon to make a clean circular cut on the anterior capsule. A percussive device would not be appropriate, which would punch ragged holes in the capsule. Thus, the frequency of the laser pulse should be in excess of 10 Hz, preferably 20 Hz - 30 Hz or more, as discussed above.

Preferably, a laser light source producing light at a wavelength readily absorbed by water is provided. Use of wavelengths that are absorbed readily by water is useful for ablation of tissues. Also, use of such wavelengths serves to prevent unwanted transmission and scatter of laser energy to adjacent or underlying tissues, resulting in minimal thermal damage to these tissues. It is also preferred that the laser light be deliverable by an optical fiber to allow the user of the probe to deliver the laser light energy by hand. Hand delivery is important for allowing delicate manipulations within the eye and other tightly confined tissues.

Thus, a preferred laser light source is an Erbium:YAG laser which produces laser light energy of wavelength 2.94 $\mu$m, a wavelength at which water has an absorbance peak. Thus, one preferred wavelength range for the light energy for use with the probe 400 is the range from 2.8 $\mu$m to 3.0 $\mu$m. The Erbium:YAG laser provides several additional advantages. First, the energy is non-ultraviolet, thereby allowing work in the eye with increased safety, obviating the need to use blocking elements or device to prevent retinal toxicity. Second, the Erbium:YAG light source can also be configured to provide the pulse frequency greater than 10 Hz needed to provide smooth cutting. Third, the high absorbance by water makes the laser safer, more controllable, and more precise. Fourth, the laser has a low thermal component, allowing for precise spatial confinement of energy deposition and reducing thermal damage and charring of intraocular tissues. Finally, the Erbium:YAG laser is relatively inexpensive to manufacture and maintain compared to certain other lasers.

Laser light sources which produce energy at other non-ultraviolet absorbance peaks of water, such as 2.1 $\mu$m, and are deliverable by optical fiber provide advantages similar to those provided by the Erbium:YAG laser. Thus, another preferred laser light source is the Holmium:YAG laser which is hand deliverable and provides laser light energy within the range of 1.9 $\mu$m to 2.2 $\mu$m.

Delivering laser light energy by fiber 318 provides the additional advantage of allowing use of the bare fiber through removal of the probe tip. Thus, in the preferred embodiment, the probe tip is removable by hand to expose the distal end of the fiber 318, Thereby enabling use of the bare fiber end from the same hand held instrument. Use of the bare fiber is advantageous in many procedures, such as the excision of a vitreous band within the eye of a mammal.

As seen in Figure 4, laser light energy exits the probe along its output axis 550 to contact the tissue at a point of contact 575. In order to view the point of contact 575 through the viewing axis 525, the surgeon or operator of the probe must look Through the probe 400. Advantageously, when the incision in the capsule 524 is made with a transparent laser probe having an uncoated, transparent reflecting surface 368, such as the probe 400 of Figure 3a, the surgeon can look through the surface 368 of the probe 400 to view the incision 560 at the point of contact 575 while the incision 560 is being cut. Thus, in order to view the point of contact 575 the surgeon can look Through the cornea 512, pupil 514 and the transparent reflecting surface 368 along the viewing axis 525. Preferably, the viewing axis 525 is generally parallel to the output axis 550, and in an especially preferred embodiment, the viewing axis 525 is collinear with the output axis 550. In many procedures, including anterior capsulotomies, it is desirable to provide a microscope (not shown) for viewing the point of contact under magnification.

Being able to view the point of contact 575 while the incision 560 is being cut, advantageously allows the surgeon to avoid sensitive areas and to more easily control the size and shape of the incision 560. Advantageously, viewing the point of contact 575 also allows the surgeon to more readily cut a smooth incision 560. Thus, decentration and other problems associated with less smooth incisions are avoided.

After the smooth incision 560 has been made on the anterior capsule, the cutout portion of the anterior capsule 524 inside the incision 560 is removed to expose the underlying lens 528. If necessary, This cut out portion of the lens 528 can be manipulated using the wedge formed by the reflecting surface 368 on the probe 400. Advantageously, this wedge can be used to manipulate other tissues inside the eye or elsewhere as well. The wedge has also been found to be advantageous in readily allowing the user of the probe to separate planes of tissue, such as in separating fascia from muscle or separating the anterior capsule 524 from the underlying lens 528 within the eye 500.

For insertion of an intraocular lens (IOL), the lens 528 can first be emulsified in a manner known to those of skill in the art, such as through phacoemulsification using an ultrasonic device. Advantageously, as shown in Figure 11, the laser probe 400 can also be used for emulsification, preferably using a higher energy level than used for incision, e.g. 100 mJ/pulse. The probe 400 can be used to deliver light energy to emulsify the lens within the completed capsular margin 600. There is less need for high frequency of laser light energy pulses during emulsification, thus frequencies of 5 Hz or less can be used. During emulsification, the high energy laser light energy exits the probe along output axis 550, and the lens tissue within the margin 600 contacted by the laser light can be viewed through the probe 400 as discussed above.

The emulsified lens material can advantageously be removed using irrigation and aspiration supplied along with the probe 400. The use of irrigation means 620 is shown in Figure 11. The use of irrigation means 620 and aspiration means for this purpose as part of a laser probe is well known, and has been described, for example in U.S. Patents Nos. 4,846,172 and 4,784,132, the disclosures of which are hereby incorporated by reference. Irrigation and/or aspiration can also be supplied as separate components, as is well known to those of skill in the art. After emulsification of the lens 528, the eye 500 is ready for implantation of the IOL.

It will be appreciated that certain structural variations may suggest themselves to those skilled in the art. The foregoing detailed description is to be clearly understood as given by way of example within the scope of the appended claims.

**Claims**

1. A laser surgery probe (200; 300; 400) that outputs a beam of laser light, comprising an optical member (262; 362) , characterised in that said optical member comprises a transparent rod (262; 362) having a longitudinal axis and having a reflecting surface (268; 368) that is inclined at an angle to said longitudinal axis of the rod for reflecting said beam of laser light out of said optical member along a single output path (550) that extends generally transversely to the longitudinal axis of the rod from said reflecting surface (268; 368) to a spot on tissue, and in that said reflecting surface (268; 368) is transparent to visible light so that visible light propagating from said spot on said tissue towards said reflecting surface (268; 368) along said output path (550) propagates through said reflecting surface so as to be visible along a viewing axis (525) that is generally transverse to the longitudinal axis of the rod (262; 362).

2. A probe according to Claim 1 wherein the laser light outputted from the probe comprises a beam having a wavelength in an invisible portion of the optical spectrum.

3. A probe according to Claim 2 wherein said laser light beam has a wavelength at an absorbance peak of water.

4. A probe according to Claim 2 or Claim 3 wherein said wavelength is 2.94 μm.

5. A probe according to any one of Claims 1 to 4 inclusive, wherein said reflecting surface (268; 368) has an index of refraction which reflects the laser light through total internal reflection.

6. A probe according to any preceding Claim wherein the optical member (262; 362) provides a probe tip that is transversely offset from the longitudinal axis , and thus adapted for manipulation of tissue.

7. A probe according to any preceding Claim wherein the transparent rod comprises sapphire.

8. A probe according to any preceding Claim wherein the probe further comprises a head element (330) having a longitudinal cavity with proximal, middle and distal sections, the proximal section being adapted to accept an optical fiber for guiding the laser light, and the distal section being adapted to accept at least a portion of the transparent rod.

9. A probe according to any preceding claim wherein the optical fiber is a fluoride-based fiber.

10. A probe according to Claim 8 or 9 wherein the probe includes a fiber holder (358) for retaining the optical fiber in the proximal section of the head element (330).

11. A probe according to any one of Claims 8 to 10 inclusive wherein the middle section comprises a lens (366) for collimating the laser light entering the transparent rod.

12. A probe according to Claim 11 wherein the lens (366) is a spherical microball.

13. A probe according to Claim 12 wherein the spherical microball has an anti-reflection coating.

## Patentansprüche

1. Chirurgische Lasersonde (200; 300; 400), die einen Laserlichtstrahl aussendet, mit einem optischen Element (262; 362), dadurch gekennzeichnet, daß das optische Element einen lichtdurchlässigen Stab (262; 362) mit einer Längsachse und einer reflektierenden Oberfläche (268; 368) aufweist, die unter einem Winkel gegen die Längsachse des Stabes geneigt ist, um den Laserlichtstrahl entlang einem einzigen Austrittsweg (550) aus dem optischen Element heraus zu reflektieren, der sich im allgemeinen quer zur Längsachse des Stabes von der reflektierenden Oberfläche (268; 368) bis zu einem Lichtfleck auf einem Gewebe erstreckt, und daß die reflektierende Oberfläche (268; 368) für sichtbares Licht durchlässig ist, so daß sichtbares Licht, das sich von dem Lichtfleck auf dem Gewebe entlang dem Austrittsweg (550) zu der reflektierenden Oberfläche (268; 368) ausbreitet, durch die reflektierende Oberfläche hindurchtritt und entlang einer Beobachtungsachse (525) sichtbar ist, die im allgemeinen quer zur Längsachse des Stabes (262; 362) gerichtet ist.

2. Sonde nach Anspruch 1, wobei der aus der Sonde austretende Laserlichtstrahl einen Strahl mit einer Wellenlänge in einem unsichtbaren Teil des optischen Spektrums aufweist.

3. Sonde nach Anspruch 2, wobei der Laserlichtstrahl eine Wellenlänge bei einem Absorptionsmaximum von Wasser aufweist.

4. Sonde nach Anspruch 2 oder Anspruch 3, wobei die Wellenlänge 2,94 μm beträgt.

5. Sonde nach einem der Ansprüche 1 bis einschließlich 4, wobei die reflektierende Oberfläche (268; 368) einen Brechungsindex aufweist, der zur Reflexion des Laserlichts durch innere Totalreflexion führt.

6. Sonde nach einem der vorstehenden Ansprüche, wobei das optische Element (262; 362) eine Sondenspitze bildet, die quer gegen die Längsachse versetzt und auf diese Weise an die Gewebemanipulation angepaßt ist.

7. Sonde nach einem der vorstehenden Ansprüche, wobei der lichtdurchlässige Stab Saphir aufweist.

8. Sonde nach einem der vorstehenden Ansprüche, wobei die Sonde ferner ein Kopfelement (330) mit einem Längshohlraum mit proximalen, mittleren und distalen Abschnitten aufweist, wobei der proximale Abschnitt für die Aufnahme einer optischen Faser zum Leiten des Laserlichts eingerichtet ist und der distale Abschnitt für die Aufnahme mindestens eines Teils des lichtdurchlässigen Stabes eingerichtet ist.

9. Sonde nach einem der vorstehenden Ansprüche, wobei die optische Faser eine Faser auf Fluoridbasis ist.

10. Sonde nach Anspruch 8 oder Anspruch 9, wobei die Sonde einen Faserhalter (358) zum Festhalten der optischen Faser im proximalen Abschnitt des Kopfelements (330) aufweist.

11. Sonde nach einem der Ansprüche 8 bis einschließlich 10, wobei der mittlere Abschnitt eine Linse (366) zum Kollimieren des in den lichtdurchlässigen Stab eintretenden Laserlichts aufweist.

12. Sonde nach Anspruch 11, wobei die Linse (366) eine sphärische Mikrokugel ist.

13. Sonde nach Anspruch 12, wobei die sphärische Mikrokugel eine Antireflexbeschichtung aufweist.

## Revendications

1. Sonde chirurgicale à faisceau laser (200; 300; 400) qui émet un faisceau de lumière laser, comprenant un élément optique (262; 362), caractérisée en ce que ledit élément optique comprend une tige transparente (262; 362) ayant un axe longitudinal et ayant une surface réfléchissante (268; 368) qui est inclinée à un certain angle par rapport audit axe longitudinal de la tige, de façon à réfléchir ledit faisceau de lumière laser vers l'extérieur dudit élément optique le long d'un seul chemin de sortie (550) qui s'étend globalement transversalement à l'axe longitudinal de la tige de ladite surface réfléchissante (268; 368) à un emplacement sur le tissu, et en ce que ladite surface réfléchissante (268; 368) est transparente à la lumière visible, si bien que la lumière visible se propageant dudit emplacement sur ledit tissu en direction de ladite surface réfléchissante (268; 368) le long dudit chemin de sortie (550) se propage à travers ladite surface réfléchissante de façon à être visible le long d'un axe de visualisation (525) qui est globalement transversal à l'axe longitudinal de la tige (262; 362).

2. Sonde selon la revendication 1, dans laquelle la lumière laser émise de la sonde comprend un faisceau ayant une longueur d'onde située dans une partie invisible du spectre optique.

3. Sonde selon la revendication 2, dans laquelle ledit faisceau de lumière laser a une longueur d'onde à un pic d'absorbance de l'eau.

**4.** Sonde selon la revendication 2 ou la revendication 3, dans laquelle ladite longueur d'onde est de 2,94 µm.

**5.** Sonde selon l'une quelconque des revendications 1 à 4 comprise, dans laquelle ladite surface réfléchissante (268; 368) a un indice de réfraction qui réfléchit la lumière laser par l'intermédiaire d'une réflexion interne totale.

**6.** Sonde selon l'une quelconque des revendications précédentes, dans laquelle l'élément optique (262; 362) présente une pointe de sonde qui est décalée transversalement de l'axe longitudinal, et adaptée ainsi pour la manipulation de tissu.

**7.** Sonde selon l'une quelconque des revendications précédentes, dans laquelle la tige transparente comprend du saphir.

**8.** Sonde selon l'une quelconque des revendications précédentes, dans laquelle la sonde comprend en outre un élément formant tête (330) possédant une cavité longitudinale dotée de sections proximale, médiane et distale, la section proximale étant conçue pour accepter une fibre optique destinée à guider la lumière laser, et la section distale étant conçue pour accepter au moins une partie de la tige transparente.

**9.** Sonde selon l'une quelconque des revendications précédentes, dans laquelle la fibre optique est une fibre à base de fluorure.

**10.** Sonde selon la revendication 8 ou 9, dans laquelle la sonde comprend un porte-fibre (358) destiné à retenir la fibre optique dans la section proximale de l'élément formant tête (330).

**11.** Sonde selon l'une quelconque des revendications 8 à 10 comprise, dans laquelle la section médiane comprend une lentille (366) destinée à collimater la lumière laser entrant dans la tige transparente.

**12.** Sonde selon la revendication 11, dans laquelle la lentille (366) est une microbille sphérique.

**13.** Sonde selon la revendication 12, dans laquelle la microbille sphérique possède un revêtement antireflet.

FIG 1

FIG. 2

FIG.3

FIG.3a

FIG.3b

EP 0 578 756 B1

FIG 4

FIG 5